# EUROPÄISCHE PATENTSCHRIFT

(11) **EP 3 676 576 B1**
(45) Veröffentlichungstag und Bekanntmachung des Hinweises auf die Patenterteilung: **28.12.2022**
(21) Anmeldenummer: 18758579.9
(22) Anmeldetag: 16.08.2018
(51) Int. Cl.: G01N 33/18, G01N 27/07, G01F 23/2962, G01F 23/296

(54) **VORRICHTUNG ZUM BESTIMMEN EINES FLUIDLEVELS UND EINER QUALITÄT EINES FLUIDS**
DEVICE FOR DETERMINING A FLUID LEVEL AND A QUALITY OF A FLUID
DISPOSITIF POUR DÉTERMINER UN NIVEAU ET UNE QUALITÉ DE FLUIDE

(30) Priorität: 31.08.2017 DE 102017215214
(43) Veröffentlichungstag der Anmeldung: 08.07.2020
(73) Patentinhaber: Vitesco Technologies Germany GmbH, 93055 Regensburg (DE)
(72) Erfinder: PFEIFFER, Karl-Friedrich, 91058 Erlangen (DE); WEBER, Claus, 90431 Nürnberg (DE)
(74) Vertreter: Vitesco Technologies
(86) Internationale Anmeldenummer: PCT/EP2018/072282
(87) Internationale Veröffentlichungsnummer: WO 2019/042788

(56) Entgegenhaltungen:
- EP-A2- 0 195 849
- WO-A1-2016/177557
- US-A1- 2009 278 699
- US-A1- 2015 013 646

## Beschreibung

Die vorliegende Erfindung betrifft eine Vorrichtung zum Bestimmen einer Qualität eines Fluids, insbesondere von deionisiertem oder demineralisiertem Wasser zur Einspritzung in eine Brennkraftmaschine.

Ultraschallsensoren werden beispielsweise zum Erfassen des Füllstands eines Öls innerhalb einer Ölwanne einer Brennkraftmaschine verwendet. Dabei werden zur Erfassung des Füllstands vom Ultraschallsensor Ultraschallwellen ausgegeben und auf Grundlage der an der Öloberfläche reflektierten Schallwellen und deren Laufzeit der Füllstand ermittelt. Hierzu weist ein Ultraschallsensor einen Ultraschallsender und -empfänger auf.

Die Elektronik des Ultraschallwandlers, die beispielsweise ein Piezo-Element und einen integrierten Schaltkreis, beispielsweise einen ASIC, umfasst, wird nach dem elektrischen Verbinden üblicherweise in einem hohlen Gehäuse angeordnet und dort ortsfest fixiert, beispielsweise mittels eines Klebers. In alternativen Ausgestaltungen ist es bekannt, dass diese Elektronik mittels einer geeigneten Einrichtung anderweitig ortsfest innerhalb des Gehäuses platziert wird, beispielsweise durch Klipsen.

Zudem ist aus der DE 10 2012 014 307 A1 ein Verfahren und eine Sensorvorrichtung zur Erfassung eines Flüssigkeitsstands für ein Fahrzeug bekannt. Die darin offenbarte Sensoreinrichtung umfasst einen Flüssigkeitsstandsensor und einen Lagesensor. Der Flüssigkeitsstandsensor und der Lagesensor sind derart in der Sensoreinrichtung angeordnet, dass der Flüssigkeitsstandsensor und der Lagesensor eine definierte räumliche Beziehung aufweisen, sodass eine von dem Lagesensor erfasste Lage auch die Lage des Flüssigkeitsstandsensors bestimmt. Die Sensoreinrichtung ist ausgestaltet, um bei dem Erfassen des Flüssigkeitsstands die räumliche Beziehung zu berücksichtigen.

Ultraschallsensoren können außerdem dazu verwendet werden, den Zustand oder die Qualität von anderen Fluiden, wie beispielsweise einer Harnstofflösung, zu ermitteln. Hierzu kann es vorteilhaft sein, dass mittels eines Temperaturfühlers die Temperatur des Fluids erfasst werden kann, die mit der mit Hilfe einer bereitgestellten Referenzstrecke ermittelten Schallgeschwindigkeit in Zusammenhang gebracht wird. Hieraus können Rückschlüsse auf den Zustand oder die Qualität des Fluids gezogen werden

Die US 2015/013646 A1 betrifft eine multifunktionale Fluidlevel- und Fluidqualitätsbestimmungsvorrichtung.

Der vorliegenden Erfindung liegt die Aufgabe zu Grunde, eine Vorrichtung zum Bestimmen eines Fluidlevels und einer Qualität eines sich in einem Fluidbehälter befindenden Fluids zu schaffen, welche insbesondere die Qualität des Fluids auf einfache, zuverlässige und genaue Weise bestimmen kann.

Diese Aufgabe wird mit einer Vorrichtung gemäß unabhängigen Anspruch 1 gelöst. Bevorzugte Ausgestaltungen sind in den Unteransprüchen angegeben.

Der vorliegenden Erfindung liegt im Wesentlichen der Gedanke zu Grunde, bei einer Vorrichtung, insbesondere einem auf Ultraschallwellentechnik basierenden Fluidlevelsensor, zusätzlich elektrisch ansteuerbare Elektroden vorzusehen, die mit dem Fluid in direkten Kontakt stehen und dazu ausgebildet sind, die elektrischen Eigenschaften des Fluids zu erfassen. Dies kann insbesondere dann bevorzugt sein, wenn es sich bei dem Fluid um eine deioinisiertes oder demineralisiertes Wasser zur Einspritzung in eine Brennkraftmaschine handelt. Dabei kann mittels der erfindungsgemäßen Vorrichtung beispielhaft bestimmt werden, ob das Wasser die im Hinblick auf seine Verwendung wünschenswerte oder notwendige Reinheit aufweist. Somit kann beispielsweise überwacht werden, ob der Vorratsbehälter bei einer Betankung mit dem korrekten Fluid betankt wird und ob dieses Fluid die vorbestimmte und gewünschte Qualität aufweist oder z. B. mit Salzen verunreinigt ist.

Folglich ist eine Vorrichtung zum Bestimmen eines Fluidlevels und einer Qualität eines sich in einem Fluidbehälter befindenden Fluids offenbart, die eine Fluidlevelbestimmungsvorrichtung zum Bestimmen des Fluidlevels mit zumindest einem Schallwandler, der zum Senden und Empfangen von Schallsignalen ausgebildet ist, eine Fluidqualitätsbestimmungsvorrichtung zum Bestimmen der Qualität des Fluids mit zumindest zwei Elektroden, die dazu ausgebildet sind, mit dem Fluid in direktem Kontakt zu stehen und elektrische Eigenschaften des Fluids zu erfassen, zumindest zwei elektrisch leitende Funktionselemente, die jeweils dazu ausgebildet sind, eine von dem Bestimmen des Fluidlevels und der Qualität des Fluids unabhängige Hauptfunktion zu erfüllen, und eine Steuereinheit aufweist, die mit der Fluidlevelbestimmungsvorrichtung und der Fluidqualitätsbestimmungsvorrichtung elektrisch verbunden und dazu ausgebildet ist, aus den Signalen der Fluidlevelbestimmungsvorrichtung das Fuidlevel und aus den von der Fluidqualitätsbestimmungsvorrichtung erfassten elektrischen Eigenschaften des Fluids die Qualität des Fluids zu bestimmen. Erfindungsgemäß sind die zumindest zwei Elektroden über die zumindest zwei Funktionselemente mit der Steuereinheit elektrisch verbunden.

Die zumindest zwei Funktionselemente sind somit dazu ausgebildet, neben ihrer Hauptfunktion die Nebenfunktion der elektrischen Verbindung zwischen den zumindest zwei Elektroden und der Steuereinheit herzustellen. Dabei ist jeder Elektrode ein Funktionselement zugeordnet, das zwischen der Steuereinheit und der jeweiligen Elektrode elektrisch zwischenverbunden ist.

Vorzugsweise ist zumindest eins der zumindest zwei Funktionselemente eine Schraube, die dazu ausgebildet ist, als Hauptfunktion zwei Gehäuseteile der Vorrichtung miteinander zu verbinden.

In einer derartigen vorteilhaften Ausgestaltung kann es bevorzug sein, dass die erfindungsgemäße Vorrichtung ferner zumindest zwei Verbindungselemente aufweist, von denen jedes jeweils dazu ausgebildet ist, eine elektrische Verbindung zwischen einer zugeordneten Elektrode und einem zugeordneten Funktionselement herzustellen. Vorzugsweise umfasst zumindest eins der zumindest zwei Verbindungselemente ein Gewinde, das dazu ausgebildet ist, mit dem als Schraube ausgebildeten Funktionselement zumindest teilweise über das Gewinde elektrisch verbunden zu werden. Dabei stellen die Schraube und das mit der Schraube verschraubte Verbindungselement zwischen der zugeordneten Elektrode und der Steuereinheit sowohl eine mechanische als auch eine elektrische Verbindung her.

In einer weiteren bevorzugten Ausführungsform weist die erfindungsgemäße Vorrichtung ferner zumindest ein elektrisches Isolationselement auf, das dazu ausgebildet ist, die zumindest zwei Elektroden derart zu halten, dass diese unter dem vorbestimmten Abstand voneinander beabstandet sind. Das zumindest eine Isolationselement ist vorzugsweise ein Kunststoffhalter, der die zumindest zwei Elektroden lagert.

Es ist außerdem bevorzugt, wenn zumindest eine der zumindest zwei Elektroden als Stabelektrode ausgebildet ist. Vorzugsweise sind die zumindest zwei Elektroden jeweils als Stabelektrode ausgebildet.

Alternativ oder zusätzlich ist zumindest eine der zumindest zwei Elektroden derart ausgebildet, dass diese einer Außenkontur eines Bereichs eines Gehäuseteils der Vorrichtung entsprechend geformt ist und somit mit dem zugeordneten Gehäuseteil der Vorrichtung zumindest teilweise mechanisch gekoppelt ist. Vorzugsweise weist ein Gehäuseteil einen im Wesentlichen als zylindrischen Vorsprung ausgebildeten Schraubdom auf, durch den hindurch das Funktionselement zumindest teilweise verläuft. Eine der zumindest zwei Elektroden ist dabei um die Außenkontur des Schraubdoms herum hülsenartig geformt.

In einer weiteren vorteilhaften Ausgestaltung sind die zumindest zwei Elektroden jeweils außerhalb eines Schallsignalpfads angeordnet sind, entlang dem sich die Schallsignale des Schallwandlers ausbreiten. Damit kann gewährleistet werden, dass die Funktion der Fluidlevelbestimmungsvorrichtung durch die Elektroden der Fluidqualtitätsbestimmungsvorrichtung gestört wird. Alternativ kann es jedoch vorteilhaft sein, wenn zumindest eine der zumindest zwei Elektroden dazu ausgebildet ist, zumindest teilweise in den Schallsignalpfad derart vorzustehen, dass diese Elektrode einen Referenzreflektor für die Bestimmung der Schallgeschwindigkeit des Fluids darstellt.

Durch das Vorsehen der Elektroden und davon separaten Funktionselementen kann die Gestaltungsfreiheit beider unterschiedlichen Elemente, die jeweils unterschiedliche Hauptfunktionen erfüllen, erhöht werden. So können die Elektroden als Stabelektroden gebildet werden, die dann mittels der separaten Funktionselemente, z. B. Schrauben, mechanisch befestigt werden. Ferner kann durch die Trennung von Elektroden und Funktionselementen eine Auswählbarkeit unterschiedlicher Materialien für die beide Elemente bereitgestellt werden. Insbesondere kann es vorteilhaft sein, für die Elektroden, die mit dem zu vermessenen Fluid, wie beispielsweise ein aggressives Fluid, in direkten Kontakt treten, ein korrosionsbeständiges Material auszuwählen, wohingegen für das Funktionselement, das z. B. eine Schraube ist, ein solches Material nicht zwingend erforderlich ist. Vielmehr kann hier für das Funktionselement ein günstigeres Material ausgewählt werden.

Weitere Aufgaben und Merkmale der vorliegenden Erfindung werden dem Fachmann durch Ausüben der vorliegenden Lehre und Betrachten der beiliegenden Zeichnungen ersichtlich, in denen:
- Fig. 1: ein schematisches Blockschaltbild einer erfindungsgemäßen Vorrichtung zum Bestimmen eines Fluidlevels und einer Qualität eines sich in einem Fluidbehälter befindenden Fluids zeigt,
- Fig. 2: eine perspektivische Ansicht einer erfindungsgemäßen Vorrichtung zum Bestimmen eines Fluidlevels eines sich in einem Fluidbehälter befindenden Fluids zeigt,
- Fig. 3: eine Draufsicht auf die zwei Elektroden der Vorrichtung der Fig. 2 zeigt, und
- Fig. 4: eine Schnittansicht durch einen Bereich der Vorrichtung der Fig. 2 zeigt,
- Fig. 5: eine Schnittansicht durch einen Bereich einer alternativen Ausgestaltung der erfindungsgemäßen Vorrichtung zeigt.

Elemente gleicher Konstruktion oder Funktion sind figurenübergreifend mit den gleichen Bezugszeichen gekennzeichnet. Aus Gründen der Übersichtlichkeit sind gegebenenfalls nicht in allen dargestellten Figuren sämtliche Elemente mit Bezugszeichen gekennzeichnet.

Im Rahmen der vorliegenden Offenbarung beschreibt der Begriff "Fluidqualität" einen ein Fluid charakterisierenden Parameter. Beispielsweise können die Schallgeschwindigkeit des Fluids, die chemische Zusammensetzung des Fluids und die elektrischen Eigenschaften des Fluids als Parameter aufgefasst werden, die die Fluidqualität charakterisieren.

Ferner beschreibt im Rahmen der vorliegenden Offenbarung der Begriff "elektrische Fluideigenschaft" einen elektrischen Parameter des Fluids, mittels dem die Fluidqualität bestimmt werden kann. Beispielsweise ist die elektrische Leitfähigkeit des Fluids als "elektrische Fluideigenschaft" zu verstehen.

Im Rahmen der vorliegenden Offenbarung beschreibt außerdem der Begriff "Funktionselement" ein Element, das dazu ausgebildet ist, zum einen eine Hauptfunktion, die von dem Bestimmen des Fluidlevels und der Qualität im Wesentlichen unabhängig ist, und zum anderen eine Nebenfunktion zu erfüllen, die zum Bestimmen des Fluidlevels und der Qualität im Wesentlichen beiträgt. Insbesondere umfasst die Nebenfunktion eines Funktionselements das Bereitstellen einer elektrisch leitenden Verbindung zwischen einer Steuereinheit und zumindest einer Elektrode einer Fluidqualitätsbestimmungsvorrichtung. Beispielsweise ist das Funktionselement eine Schraube, die in ihrer Hauptfunktion zum Verbinden von zwei Komponenten, wie z. B. dem Anbringen der Elektrode der Fluidqualitätsbestimmungsvorrichtung an einem Gehäuse der erfindungsgemäßen Vorrichtung, und in ihrer Nebenfunktion zum elektrischen Verbinden der Elektrode mit der Steuereinheit ausgebildet ist.

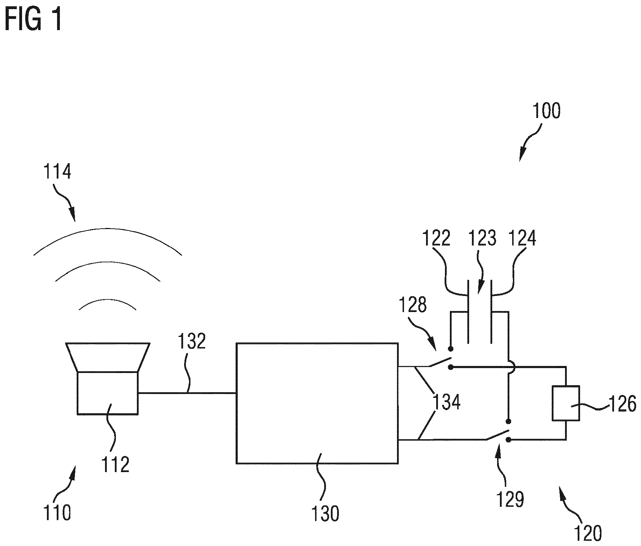

Die Fig. 1 zeigt ein schematisches Blockschaltbild einer erfindungsgemäßen Vorrichtung zum Bestimmen eines Fluidlevels und einer Qualität eines sich in einem Fluidbehälter (nicht explizit dargestellt) befindenden Fluids. Die Vorrichtung 100 umfasst eine Fluidlevelbestimmungsvorrichtung 110 mit zumindest einem Schallwandler 112, der zum Senden und Empfangen von Schallsignalen 114 ausgebildet ist. Die vom Schallwandler 112 ausgesandten Schallsignale 114 sind insbesondere Ultraschallwellen, die dazu ausgebildet sind, an einer Oberfläche des Fluids reflektiert zu werden, um wiederum dann vom Schallwandler 112 empfangen zu werden. Alternativ können zum Aussenden und Empfangen separate Schallwandler 112 vorgesehen werden. Aus der Laufzeitmessung der Schallsignale 114 des Schallwandlers 112 kann der Fluidlevel bestimmt werden.

Die Vorrichtung weist ferner eine Steuereinheit 130 auf, die über eine elektrische Verbindungsleitung 132 mit dem Schallwandler 112 verbunden und dazu ausgebildet ist, aus der Laufzeitmessung der Schallsignale 114 das Fluidlevel des Fluids zu bestimmen.

Die erfindungsgemäße Vorrichtung 100 weist ferner eine Fluidqualitätsbestimmungsvorrichtung 120 auf, die zumindest zwei Elektroden 122, 124 und einen temperaturabhängigen Widerstand 126 aufweist, die jeweils wahlweise über eine Auswahlvorrichtung, die zwei Schaltvorrichtungen 128, 129 umfasst, mit der Steuereinheit 130 elektrisch verbunden werden können. Hierfür sind elektrische Verbindungsleitungen 134 vorgesehen.

Die zumindest zwei Elektroden 122, 124 sind derart angeordnet, dass diese in direkten Kontakt mit den Fluid stehen können und voneinander unter einem vorbestimmten Abstand derart beabstandet sind, dass diese dazwischen eine Messstrecke 123 definieren. Beispielsweise bilden die beiden Elektroden 122, 124 einen Kondensator, wobei das sich dazwischen befindliches Fluid als Dielektrikum wirkt und folglich unter Kenntnis des Abstands der beiden Elektroden 122, 124 zueinander die Dielektrizitätszahl des Fluids bestimmbar ist. Mit dieser Anordnung kann auch die elektrische Leitfähigkeit des Fluids bestimmt werden. Beide elektrischen Eigenschaften korrelieren jeweils mit der Qualität des Fluids. Beispielweise sind die Elektroden 122, 124 unter einem Abstand zwischen ungefähr 2 mm und ungefähr 10 mm voneinander beabstandet und können mit einer Spannung von ungefähr 5 V beaufschlagt werden.

Der temperaturabhängige elektrische Widerstand 126 ist dazu ausgebildet, die Temperatur des Fluids zu ermitteln. Die durch den temperaturabhängigen elektrischen Widerstand 126, der beispielsweise ein NTC oder PTC sein kann, ermittelte Fluidtemperatur kann für die Bestimmung der Qualität des Fluids herangezogen werden. Alternativ oder zusätzlich kann die mittels des temperaturabhängigen elektrischen Widerstands 126 erfasste Temperatur bei der Ermittlung und/oder Auswertung der Schallgeschwindigkeit des Fluids berücksichtigt werden. Hierzu weist die Fluidlevelbestimmungsvorrichtung eine Referenzstrecke mit zumindest einem Referenzreflektor auf, die eine bekannte Laufstrecke für die Ultraschallsignale 114 bereitstellt, woraus die auch für die Fluidlevelbestimmung benötigte Schallgeschwindigkeit ermittelt werden kann.

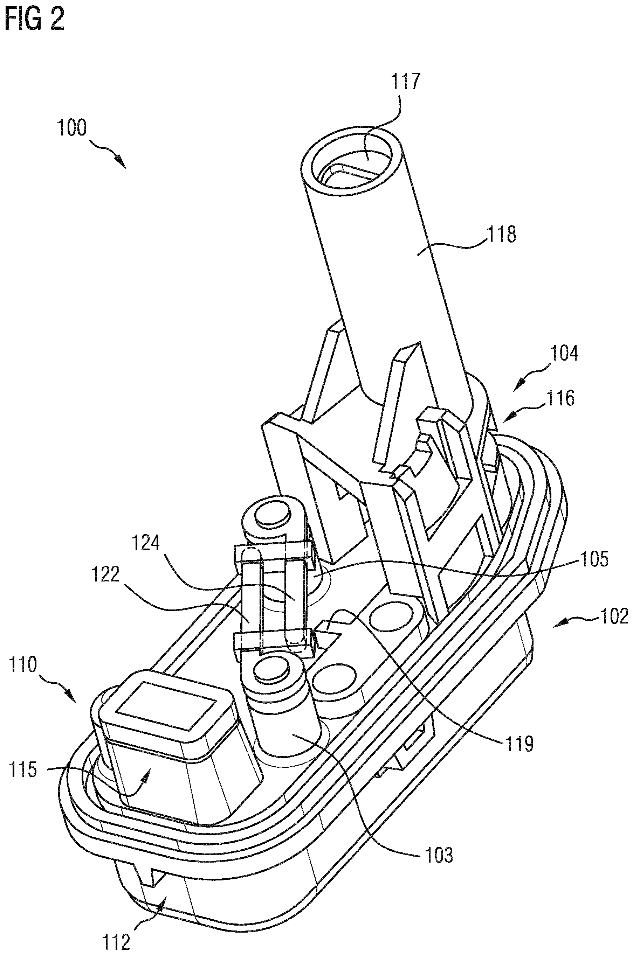

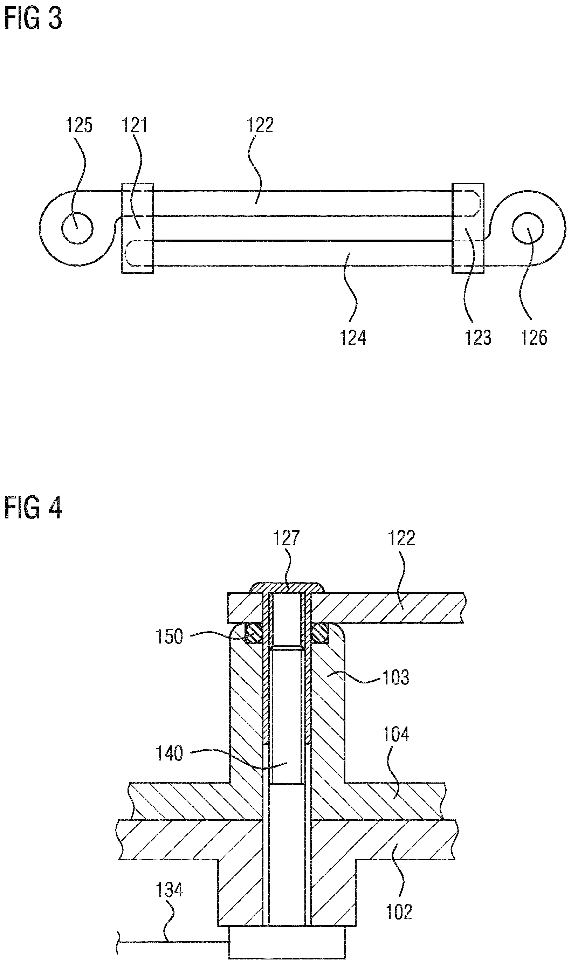

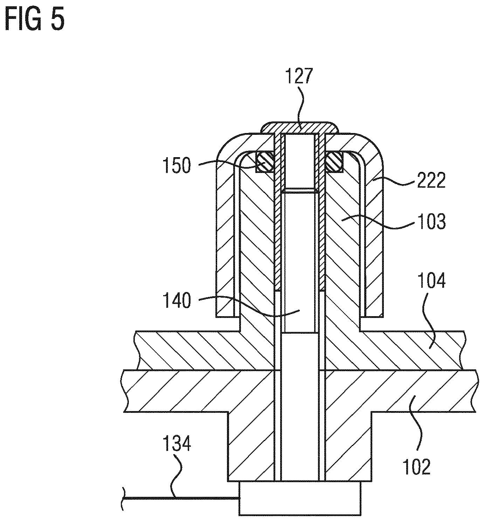

Die Fig. 2 zeigt eine perspektivische Ansicht einer beispielhaften Ausgestaltung einer erfindungsgemäßen Vorrichtung 100. Die Vorrichtung 100 weist ein Gehäuseunterteil 102 und ein Gehäuseoberteil 104 auf, die miteinander mittels Schrauben 140 (siehe Fig. 4 und 5) verbunden sind. Die Schrauben 140 sind im Sinne der vorliegenden Erfindung als Funktionselemente zu betrachten. Die Vorrichtung 100 ist dazu ausgebildet, von unten in eine Öffnung im Fluidbehälter (nicht dargestellt) derart eingesetzt zu werden, dass im Wesentlichen das gesamte Gehäuseoberteil 104 im Fluidbehälter angeordnet ist und folglich in direkten Kontakt mit dem Fluid steht. Die Schallsignale 114 werden in bekannter Weise an zwei Reflektoren 115, 116 in Richtung der Fluidoberfläche zweifach umgelenkt. Wie in der Fig. 2 gezeigt ist der Reflektor 117 mit einem Beruhigungsrohr 118 gekoppelt, das dazu ausgebildet ist, das sich darin befindliche Fluid zumindest teilweise zu beruhigen, so dass eine zuverlässige Ermittlung des Fluidlevels ermöglicht ist. Der Referenzreflektor 117 ist dafür vorgesehen und ausgebildet, auch bei starker Fahrzeugneigung ein Echosignal zu liefern, wenn der Füllstand größer oder gleich der Länge des Beruhigungsrohrs 118 ist.

Die Fluidlevelbestimmungsvorrichtung 110 weist, wie bereits erwähnt, zumindest einen Referenzreflektor 119 an bekannter Position auf, der zur Ermittlung der Schallgeschwindigkeit des Fluids in bekannter Weise dient.

Erfindungsgemäß ist nun die Fluidqualitätsbestimmungsvorrichtung 120 vorgesehen, die zumindest zwei außerhalb des Schallsignalpfads angeordnete Elektroden 122, 124 aufweist, die dazu ausgebildet sind, zumindest eine elektrische Eigenschaft des Fluids zu bestimmen, woraus dann von der Steuereinheit 130 eine Qualität des Fluids ermittelt werden kann. Die beiden Elektroden 122, 124 sind dazu ausgebildet, derart angeordnet zu werden, dass diese in direktem Kontakt mit dem Fluid stehen.

Unter zusätzlichen Verweis auf die Fig. 3 ist dargestellt, dass die zumindest zwei Elektroden 122, 124 von zwei Isolationselementen 121, 123, die beispielsweise aus Kunststoff gebildet sind, unter einem vorbestimmten Abstand zueinander gehalten werden. Die Anbindung der zumindest zwei Elektroden 122, 124 am Gehäuseoberteil 104 kann, wie in den Fig. 2, 3 und 4 gezeigt, derart erfolgen, dass bereits bestehende, im Wesentlichen als zylindrische Vorsprünge ausgebildete Schraubdome 103, 105 als Befestigungspunkt für die zumindest zwei Elektroden 122, 124 dienen. Wie der Fig. 3 zu entnehmen ist, weisen die beiden Elektroden 122, 124 jeweils Aufnahmeöffnungen 125, 126 auf, die dazu ausgebildet sind, jeweils eine elektrisch leitende Gewindehülse 127 aufzunehmen (siehe Fig. 4).

Die Fig. 4 zeigt eine Schnittansicht durch den Schraubdom 103, mit dem die Elektrode 122 mittels einer elektrisch leitenden Gewindehülse 127, die ein Verbindungselement darstellt, und mittels einer Schraube 140, die ein Funktionselement darstellt, gekoppelt ist. Bekannt ist es, das Gehäuseunterteil 102 mit dem Gehäuseoberteil 104 mittels zumindest einer Schraube 140 zu verbinden, wobei die Schraubdome im Stand der Technik lediglich eine gewindefreie Sackloch-Bohrung aufweisen, in die die selbstschneidende Schraube 140 zur Verbindung der beiden Gehäuseteile 102, 104 eingeschraubt werden.

Erfindungsgemäß werden können die bereits vorhandenen Schraubdome 103, 105 derart modifiziert werden, dass die Bohrungen nun durchgängig sind, wobei die Gewindehülse 127 ein Innengewinde aufweist, in das die Schraube 140 zur festen Verbindung der Gehäuseteile 102, 104 geschraubt werden kann. Zur sicheren Abdichtung des Gehäuseinneren kann eine Dichtung 150 vorgesehen werden. Die Schraube 140, die dazu ausgebildet ist, elektrisch leitfähig zu sein, ist dann mittels der elektrischen Verbindungsleitung 134 mit der Steuereinheit 130 elektrisch verbunden.

Folglich kann zwischen der Elektrode 122 und der Steuereinheit 130 über die elektrisch leitende Gewindehülse 127, der elektrisch leitenden Schraube 140, die das Funktionselement beschreibt, und der elektrisch leitenden Verbindungleitung 134 eine elektrische Verbindung zur Ansteuerung der Elektrode 122 hergestellt werden. In gleicher Weise erfolgt die elektrische Anbindung der Elektrode 124 am Schraubdom 105 mit einer entsprechend vorgesehenen Schraube 140 und einer entsprechend vorgesehenen Gewindehülse 127. Dabei erfüllen die als Funktionselemente dienenden Schrauben 140 als Hauptfunktion das Verbinden der Gehäuseteile 102, 104 bzw. das Anbringen der Elektroden 122, 124 am Gehäuseteil 104 (bzw. am Schraubdom 103) und als Nebenfunktion das elektrische Verbinden der Steuereinheit 130 mit den Elektroden 122, 124.

Durch die in den Fig. 2, 3 und 4 gezeigten zwei Elektroden 122, 124, die beispielsweise als Stabelektroden ausgebildet sein können, kann eine Fluidqualitätsbestimmungsvorrichtung 120 in einfacher Weise bereitgestellt werden, wobei die zumindest zwei Elektroden 122, 124 dazu ausgebildet sind, mit dem Fluid in direktem Kontakt zu stehen. Alternativ zur Anbindung der Elektroden 122, 124 über die bereits vorhandenen Schraubdome 103, 105 kann jegliches weitere Funktionselement verwendet werden, das neben seiner vorbestimmten Hauptfunktion dazu ausgebildet ist, eine elektrische Anbindung an die Elektroden 122, 124 bereitzustellen.

Die Fig. 5 zeigt eine alternative Ausgestaltung der Elektroden 122, 124. Wie der Fig. 5 entnommen werden kann, ist die Elektrode 122 nach dem Verbinden mit dem Schraubdom 103 gemäß der bereits in der Fig. 4 gezeigte Kopplungsmöglichkeit um den zugeordneten Schraubdom 103 herum angeformt. Beispielsweise kann die Elektrode aus einem geeigneten Blech in eine zum Schraubdom 103, 105 passende Form tiefgezogen werden oder auch einstückig mit der Gewindehülse 127 hergestellt werden, zum Beispiel durch ein spanendes Verfahren. Beispielweise sind die Elektroden 122, 124 der Fig. 5 unter einem Abstand zwischen ungefähr 15 mm und ungefähr 30 mm voneinander beabstandet und können mit einer Spannung von ungefähr 5 V beaufschlagt werden.

Die Ausgestaltung der Elektrode 122 der Fig. 5 weist gegenüber der Ausgestaltung der Elektrode 122 der Fig. 4 den Vorteil auf, dass diese im Hinblick auf mechanische Stabilität deutlich robuster ist und somit der vorbestimmte Abstand zwischen den Elektroden 122, 124 noch zuverlässiger auf einen vorbestimmten Wert eingestellt werden kann. Zudem ist die Gefahr einer Beschädigung der Elektrode nach Fig. 5 durch Einfrieren des Fluids oder durch im Fluid schwimmende Eisstücke wesentlich geringer. Auch der Fertigungsaufwand kann geringer sein, da die Isolationselemente 121, 123 entfallen.

## Patentansprüche

1. Vorrichtung (100) zum Bestimmen eines Fluidlevels und einer Qualität eines sich in einem Fluidbehälter befindenden Fluids, umfassend:
- eine Fluidlevelbestimmungsvorrichtung (110) zum Bestimmen des Fluidlevels mit zumindest einem Schallwandler (112), der zum Senden und Empfangen von Schallsignalen (114) ausgebildet ist,
- eine Fluidqualitätsbestimmungsvorrichtung (114) zum Bestimmen der Qualität des Fluids mit zumindest zwei Elektroden (122, 124), die dazu ausgebildet sind, mit dem Fluid in direktem Kontakt zu stehen und elektrische Eigenschaften des Fluids zu erfassen,
- zumindest zwei elektrisch leitende Funktionselemente (140), die jeweils dazu ausgebildet sind, eine von dem Bestimmen des Fluidlevels und der Qualität des Fluids unabhängige Hauptfunktion zu erfüllen, und
- eine Steuereinheit (130), die mit der Fluidlevelbestimmungsvorrichtung (110) und der Fluidqualitätsbestimmungsvorrichtung (120) elektrisch verbunden und dazu ausgebildet ist, aus den Signalen der Fluidlevelbestimmungsvorrichtung (110) das Fuidlevel und aus den von der Fluidqualitätsbestimmungsvorrichtung (120) erfassten elektrischen Eigenschaften des Fluids die Qualität des Fluids zu bestimmen, wobei die zumindest zwei Elektroden (122, 124) über die zumindest zwei Funktionselemente (140) mit der Steuereinheit (130) elektrisch verbunden sind,
wobei die zumindest zwei elektrisch leitenden Funktionselemente (140) ausgebildet sind, um, eine Hauptfunktion, die im Wesentlichen unabhängig von der Bestimmung des Fluidlevels und der Qualität ist, und eine Nebenfunktion, die im Wesentlichen zur Bestimmung des Fluidlevels und der Qualität beiträgt; wobei die Hauptfunktion der zwei elektrisch leitenden Funktionselemente (140) darin besteht, eine Verbindung zwischen mindestens einer der zumindest zwei Elektroden (122,124) der Vorrichtung (100) und einem Gehäuse der Vorrichtung (100) bereitzustellen, und wobei die Nebenfunktion der zwei elektrisch leitenden Funktionselemente (140) darin besteht, eine elektrisch leitende Verbindung zwischen der Steuereinheit (130) und mindestens einer der zumindest zwei Elektroden (122,124) der Vorrichtung (100) zur Bestimmung der Fluidqualität herzustellen.

2. Vorrichtung (100) nach Anspruch 1, wobei zumindest eins der zumindest zwei Funktionselemente eine Schraube (140) ist, die dazu ausgebildet ist, als Hauptfunktion zwei Gehäuseteile (102, 104) der Vorrichtung (100) miteinander zu verbinden.

3. Vorrichtung (100) nach Anspruch 2, ferner mit:
- zumindest zwei Verbindungselemente (127), von denen jedes jeweils dazu ausgebildet ist, eine elektrische Verbindung zwischen einer zugeordneten Elektrode (122, 124) und einem zugeordneten Funktionselement (140) herzustellen.

4. Vorrichtung (100) nach Anspruch 3, wobei zumindest eins der zumindest zwei Verbindungselemente ein Gewinde aufweist, das dazu ausgebildet ist, mit dem als Schraube (140) ausgebildeten Funktionselement zumindest teilweise über das Gewinde elektrisch verbunden zu werden.

5. Vorrichtung (100) nach einem der vorhergehenden Ansprüche, ferner mit zumindest einem elektrischen Isolationselement (121, 123), das dazu ausgebildet ist, die zumindest zwei Elektroden (122, 124) derart zu halten, dass diese unter dem vorbestimmten Abstand voneinander beabstandet sind.

6. Vorrichtung (100) nach einem der vorhergehenden Ansprüche, wobei zumindest eine der zumindest zwei Elektroden (122, 124) als Stabelektrode ausgebildet ist.

7. Vorrichtung (100) nach einem der vorhergehenden Ansprüche, wobei zumindest eine der zumindest zwei Elektroden (122, 124) derart ausgebildet ist, dass diese einer Außenkontur eines Bereichs eines Gehäuseteils (102, 102) der Vorrichtung (100) entsprechend geformt ist und somit mit dem zugeordneten Gehäuseteil (102, 104) der Vorrichtung (100) zumindest teilweise mechanisch gekoppelt ist.

8. Vorrichtung (100) nach Anspruch 7, wobei das Gehäuseteil (102, 104) einen im Wesentlichen als zylindrischen Vorsprung ausgebildeten Schraubdom (103, 105) aufweist, durch den hindurch das Funktionselement (140) zumindest teilweise verläuft, wobei eine der zumindest zwei Elektroden (122, 124) um den Schraubdom (103, 105) herum hülsenartig geformt ist.

9. Vorrichtung (100) nach einem der vorhergehenden Ansprüche, wobei die zumindest zwei Elektroden (122, 124) jeweils außerhalb einer Schallsignalpfads angeordnet sind, entlang dem sich die Schallsignale (114) des Schallwandlers (112) ausbreiten.

10. Vorrichtung (100) nach einem der vorhergehenden Ansprüche,
wobei das Fluid deionisiertes oder demineralisiertes Wasser ist, welches zur Einspritzung in eine Brennkraftmaschine ausgebildet ist,
wobei die Fluidqualitätsbestimmungsvorrichtung (120) dazu ausgebildet ist, die Reinheit des Wassers zu bestimmen.

## Claims

1. Device (100) for determining a fluid level and a quality of a fluid located in a fluid container, comprising:
- a fluid level determination device (110) for determining the fluid level with at least one sound transducer (112) which is designed for transmitting and receiving sound signals (114),
- a fluid quality determination device (114) for determining the quality of the fluid with at least two electrodes (122, 124) which are designed to be in direct contact with the fluid and to detect electrical properties of the fluid,
- at least two electrically conductive functional elements (140) which are each designed to perform a primary function that is independent of the determination of the fluid level and of the quality of the fluid, and
- a control unit (130) which is electrically connected to the fluid level determination device (110) and the fluid quality determination device (120) and is designed to determine the fluid level from the signals of the fluid level determination device (110) and the quality of the fluid from the electrical properties of the fluid detected by the fluid quality determination device (120), wherein the at least two electrodes (122, 124) are electrically connected to the control unit (130) via the at least two functional elements (140),
wherein the at least two electrically conductive functional elements (140) are designed to perform a primary function, which is substantially independent of the determination of the fluid level and of the quality, and a secondary function, which substantially contributes to the determination of the fluid level and of the quality; wherein the primary function of the two electrically conductive functional elements (140) consists in providing a connection between at least one of the at least two electrodes (122, 124) of the device (100) and a housing of the device (100), and wherein the secondary function of the two electrically conductive functional elements (140) consists in establishing an electrically conductive connection between the control unit (130) and at least one of the at least two electrodes (122, 124) of the device (100) for determining the fluid quality.

2. Device (100) according to Claim 1, wherein at least one of the at least two functional elements is a screw (140) which is designed to connect two housing parts (102, 104) of the device (100) to each other as the primary function.

3. Device (100) according to Claim 2, further having:
- at least two connection elements (127), each of which is respectively designed to establish an electrical connection between an assigned electrode (122, 124) and an assigned functional element (140).

4. Device (100) according to Claim 3, wherein at least one of the at least two connection elements has a thread which is designed to be electrically connected, at least partially via the thread, to the functional element designed as a screw (140).

5. Device (100) according to one of the preceding claims, further having at least one electrical insulation element (121, 123) which is designed to hold the at least two electrodes (122, 124) in such a way that they are spaced apart from each other by the predetermined spacing.

6. Device (100) according to one of the preceding claims, wherein at least one of the at least two electrodes (122, 124) is designed as a rod electrode.

7. Device (100) according to one of the preceding claims, wherein at least one of the at least two electrodes (122, 124) is designed in such a way that it is shaped in accordance with an outer contour of a region of a housing part (102, 102) of the device (100) and is thus at least partially mechanically coupled to the assigned housing part (102, 104) of the device (100).

8. Device (100) according to Claim 7, wherein the housing part (102, 104) has a screw boss (103, 105) which is designed substantially as a cylindrical projection and through which the functional element (140) at least partially runs, wherein one of the at least two electrodes (122, 124) is shaped around the screw boss (103, 105) in the manner of a sleeve.

9. Device (100) according to one of the preceding claims, wherein the at least two electrodes (122, 124) are each arranged outside of a sound signal path along which the sound signals (114) of the sound transducer (112) propagate.

10. Device (100) according to one of the preceding claims,
wherein the fluid is deionized or demineralized water which is designed for injection into an internal combustion engine,
wherein the fluid quality determination device (120) is designed to determine the purity of the water.

## Revendications

1. Dispositif (100) permettant de déterminer un niveau de fluide et une qualité d'un fluide se trouvant dans un réservoir de fluide, comprenant :
- un dispositif de détermination de niveau de fluide (110) pour déterminer le niveau de fluide à l'aide d'au moins un transducteur acoustique (112) qui est réalisé pour émettre et recevoir des signaux acoustiques (114),
- un dispositif de détermination de qualité de fluide (114) pour déterminer la qualité du fluide à l'aide d'au moins deux électrodes (122, 124) qui sont réalisées pour être en contact direct avec le fluide et pour détecter des propriétés électriques du fluide,
- au moins deux éléments fonctionnels (140) électriquement conducteurs qui sont respectivement réalisés pour remplir une fonction principale indépendante de la détermination du niveau de fluide et de la qualité du fluide, et
- une unité de commande (130) qui est reliée électriquement au dispositif de détermination de niveau de fluide (110) et au dispositif de détermination de qualité de fluide (120), et qui est réalisée pour déterminer le niveau de fluide à partir des signaux du dispositif de détermination de niveau de fluide (110), et pour déterminer la qualité du fluide à partir des propriétés électriques du fluide, détectées par le dispositif de détermination de qualité de fluide (120),
dans lequel les au moins deux électrodes (122, 124) sont reliées électriquement à l'unité de commande (130) par l'intermédiaire des au moins deux éléments fonctionnels (140),
dans lequel les au moins deux éléments fonctionnels (140) électriquement conducteurs sont réalisés pour remplir une fonction principale qui est substantiellement indépendante de la détermination du niveau de fluide et de la qualité, et une fonction auxiliaire qui contribue substantiellement à la détermination du niveau de fluide et de la qualité ; la fonction principale des deux éléments fonctionnels (140) électriquement conducteurs consistant à fournir une liaison entre au moins l'une des au moins deux électrodes (122, 124) du dispositif (100) et un boîtier du dispositif (100), et la fonction auxiliaire des deux éléments fonctionnels (140) électriquement conducteurs consistant à établir une liaison électriquement conductrice entre l'unité de commande (130) et au moins l'une des au moins deux électrodes (122, 124) du dispositif (100) afin de déterminer la qualité de fluide.

2. Dispositif (100) selon la revendication 1, dans lequel au moins l'un des au moins deux éléments fonctionnels est une vis (140) qui est réalisée pour relier comme fonction principale deux parties de boîtier (102, 104) du dispositif (100) l'une à l'autre.

3. Dispositif (100) selon la revendication 2, comprenant en outre :
- au moins deux éléments de liaison (127) dont chacun est respectivement réalisé pour établir une liaison électrique entre une électrode (122, 124) associée et un élément fonctionnel (140) associé.

4. Dispositif (100) selon la revendication 3, dans lequel au moins l'un des au moins deux éléments de liaison présente un filetage qui est réalisé pour être au moins partiellement relié électriquement à l'élément fonctionnel réalisé sous forme de vis (140) par l'intermédiaire du filetage.

5. Dispositif (100) selon l'une quelconque des revendications précédentes, comprenant en outre au moins un élément d'isolation électrique (121, 123) qui est réalisé pour maintenir les au moins deux électrodes (122, 124) de telle sorte qu'elles sont espacées les unes des autres à une distance prédéterminée.

6. Dispositif (100) selon l'une quelconque des revendications précédentes, dans lequel au moins l'une des au moins deux électrodes (122, 124) est réalisée sous forme d'électrode en baguette.

7. Dispositif (100) selon l'une quelconque des revendications précédentes, dans lequel au moins l'une des au moins deux électrodes (122, 124) est réalisée de telle sorte qu'elle est formée selon un contour extérieur d'une zone d'une partie de boîtier (102, 102) du dispositif (100) et est donc couplée au moins partiellement de manière mécanique à la partie de boîtier (102, 104) associé du dispositif (100).

8. Dispositif (100) selon la revendication 7, dans lequel la partie de boîtier (102, 104) présente un bossage pour vis (103, 105), réalisé substantiellement sous la forme d'une saillie cylindrique, à travers lequel l'élément fonctionnel (140) s'étend au moins partiellement, dans lequel l'une des au moins deux électrodes (122, 124) est formée à la manière d'une douille autour du bossage pour vis (103, 105).

9. Dispositif (100) selon l'une quelconque des revendications précédentes, dans lequel les au moins deux électrodes (122, 124) sont respectivement disposées en dehors d'un chemin de signal acoustique le long duquel se propagent les signaux acoustiques (114) du transducteur acoustique (112).

10. Dispositif (100) selon l'une quelconque des revendications précédentes,
dans lequel le fluide est de l'eau désionisée ou déminéralisée qui est réalisée pour être injectée dans un moteur à combustion interne,
dans lequel le dispositif de détermination de qualité de fluide (120) est réalisé pour déterminer la pureté de l'eau.
